# DEMANDE DE BREVET EUROPEEN

(11) **EP 4 400 176 A1**
(43) Date de publication de la demande: **17.07.2024**
(21) Numéro de dépôt: 23219850.7
(22) Date de dépôt: 22.12.2023
(51) Int. Cl.: A61Q 1/06, A61K 8/92, A61K 8/36

(54) **COMPOSITION COSMÉTIQUE COMPRENANT UN MÉLANGE D HUILES VÉGÉTALES ISSU D'UN TRAITEMENT PLASMA, SON PROCÉDÉ D OBTENTION ET UTILISATION**

(30) Priorité: 23.12.2022 EP 22290083
(71) Demandeur: Green Frix, 7522 Tournai (BE); Chanel Parfums Beauté, 92200 Neuilly-sur-Seine (FR)
(72) Inventeur: DANNEAUX, Frédéric, 1380 Lasne (BE); LE BIHAN, Sophie, 59390 Toufflers (FR)
(74) Mandataire: Calysta NV

(57) **Abrégé**

Une composition comprenant :
- un mélange d'au moins deux huiles végétales traitées ensemble selon un procédé plasma dans une teneur comprise entre 20% et 99% en poids,
- lesdites au moins deux huiles végétales étant choisies parmi :
∘ une huile végétale (H1) comprenant :
▪ au moins un triglycéride constitué d'esters d'acides gras et de glycérol, ou
▪ au moins un ester d'acide gras polyinsaturé, ou
▪ leur(s) mélange(s),

∘ une huile (H2) comprenant :
▪ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
▪ au moins un acide gras monoinsaturé, ou
▪ au moins un diester d'alkyle dont au moins une teneur de 90% en poids d'alkyle(s) linéaire(s) monoinsaturé(s) ou saturé(s), ou
▪ leur(s) mélange(s).

## Description

### Domaine technique

La présente divulgation relève du domaine de la formulation cosmétique et plus particulièrement des compositions cosmétiques comprenant au moins un mélange d'huiles issu d'un procédé plasma, conférant une persistance accrue d'au moins un effet cosmétique, notamment de maquillage et/ou de soin apporté après application de la composition, ainsi qu'une amélioration de la tenue de la composition après application sur les matières kératiniques.

De telles huiles permettent de conférer à ladite composition cosmétique des propriétés de longue tenue ainsi qu'une homogénéité à l'application. Les huiles ayant subi un traitement plasma ont une augmentation de leur viscosité permettant ainsi de pouvoir les utiliser seules dans des compositions cosmétiques de type rouge à lèvres liquide, aussi dénommé gloss, ou de type rouge à lèvres solide, dénommé stick, sans ajout de gélifiant ou polymères structurants. La composition cosmétique présente alors de bonne propriété d'adhésion permettant de garantir sa tenue sans impacter la brillance recherchée notamment dans les produits de maquillage.

### Technique antérieure

Les compositions cosmétiques de maquillage ou de soin sont couramment employées pour apporter un aspect esthétique lors de l'application sur la peau et les lèvres, cet effet devant perdurer au cours du temps. En particulier, dans le cas des produits de maquillage pour les lèvres, sous forme liquide, semi-fluide ou solide (stick), on cherche à avoir une bonne tenue dans le temps de la couleur et de la brillance, et une bonne résistance au transfert. Mais il est difficile de combiner les propriétés de brillance et de tenue au sein d'une seule et même composition. En effet, pour apporter de la structure à ces compositions on utilise généralement une combinaison d'huile, et de gélifiant voire de cire dans le cas des compositions de type solide ou semi-solide. Or ces gélifiants ont tendance à matifier le résultat maquillage et diminuer ainsi le côté brillant naturel apporté par les huiles.

Il est connu de l'art antérieur, par exemple, pour réaliser des compositions de maquillage liquide des lèvres de type gloss, des produits en double application, avec l'application d'une première composition permettant d'apporter la couleur et la tenue de celui-ci, suivi de l'application d'une seconde composition ayant pour objectif de déposer un film brillant.

Il est aussi connu de l'art antérieur pour obtenir par exemple des compositions de maquillage solide des lèvres, de type stick, d'utiliser comme corps blanc, c'est-à-dire, le corps structurant de la composition, une composition comprenant un mélange d'huiles, de cires, de beurres, de polymères pâteux, et de gélifiants. La présence dans le corps blanc d'un mélange d'huiles et de cires n'est pas suffisant à lui-seul pour apporter de la tenue au résultat maquillage car il est trop gras. Les beurres, et/ou polymères pâteux, et/ou gélifiants vont permettre d'apporter les propriétés d'adhésion et de collant nécessaire pour permettre une tenue du maquillage. De tels ingrédients sont généralement issus de la pétrochimie et sont souvent des dérivés siliconés. Dans l'objectif d'avoir des compositions plus naturelles et respectueuses de l'environnement il est souhaitable de trouver des alternatives à la substitution voire l'élimination de ces ingrédients au sein des compositions cosmétiques.

De plus, les agents permettant d'apporter une structure à la composition, confèrent une opacité au milieu : les cires, les beurres, les sels d'aluminium, les polymères de type PVP/Eicosène copolymère, apportent de la viscosité mais aussi une opacité au milieu, entraînant une perte de transparence et de brillance de la composition cosmétique.

Il serait donc souhaitable de rechercher des solutions techniques à la fois plus respectueuses de l'environnement, en limitant l'utilisation d'ingrédients issus de la pétrochimie et de dérivés siliconés, tout en alliant un résultat maquillage de tenue et de brillance.

La présente invention vise donc à proposer une solution à ces différents problèmes, et à proposer la mise en oeuvre d'un mélange d'huiles végétales traité par un procédé plasma, en tant qu'agent structurant principal dans des compositions liquides ou en combinaison avec au moins une cire dans des compositions cosmétiques solides ou semi-solides.

### Description détaillée de l'invention

La présente invention concerne donc une composition cosmétique de maquillage et/ou de soin de la peau et/ou des lèvres, procurant un dépôt de maquillage ou de soin présentant une persistance dans le temps améliorée, permettant ainsi une meilleure tenue dans le temps de sa couleur et/ou de sa brillance. Plus spécifiquement, la présente divulgation concerne une composition cosmétique mettant en oeuvre, en tant qu'ingrédient principal, un mélange d'huiles végétales issu d'un traitement plasma.

Le traitement plasma est opéré en faisant appel à un plasmagène constitué d'hydrogène. Le traitement plasma est de préférence exempt de précurseur. Ainsi, le passage du mélange d'huiles d'origine végétale a lieu dans une enceinte mise sous vide dans laquelle le plasma est généré, de préférence au moyen d'une décharge à barrière diélectrique de type DBD (« Dielectric Barrier Discharge », en anglais). La décharge peut être obtenue par l'utilisation d'au moins 5 électrodes mises en parallèle les unes par rapport aux autres, de préférence au moins 15 électrodes, plus préférentiellement au moins 30 électrodes.

La présente invention a ainsi pour objet, selon un premier aspect, une composition cosmétique comprenant :
- un mélange d'au moins deux huiles végétales traitées ensemble selon un procédé plasma dans une teneur comprise entre 20% et 99% en poids, par rapport au poids total de la composition,
- ledit mélange présentant une viscosité dynamique à 50°C supérieure à 250 mPa.s, préférentiellement supérieure à 500 mPa.s ou supérieure à 2000 mPa.s, préférentiellement supérieure à 3000 mPa.s, et inférieure à 10 000 mPa.s ou une viscosité cinématique à 50°C comprise entre 250 et 20000 mm²/sec,

- les au moins deux huiles végétales étant choisies parmi :
   - au moins une huile végétale H1 comprenant :
      ∘ au moins un triglycéride formé par une molécule de glycérol et 3 molécules d'acide gras, ou
      ∘ au moins un ester d'acide gras polyinsaturé, ou
      ∘ leur(s) mélange(s),
   - au moins une huile H2 comprenant :
      ∘ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
      ∘ au moins un acide gras monoinsaturé, ou
      ∘ au moins un diester d'alkyle dont au moins une teneur de 90% en poids d'alkyle(s) linéaire(s) monoinsaturé(s) ou saturé(s), ou
      ∘ leur(s) mélange(s).

Il est en effet du mérite de la demanderesse d'avoir mis en évidence que l'utilisation d'un mélange d'huiles végétales ayant préalablement subi un traitement par un procédé plasma, permettait non seulement d'augmenter la viscosité du mélange ainsi obtenu, et ainsi de limiter l'utilisation au sein de la composition cosmétique le comprenant d'ingrédient tels que des gélifiants ou des polymères structurants, mais conférer également des propriétés de tenue et de brillance au produit cosmétique tout en renforçant sa stabilité structurelle.

Ainsi lors du traitement plasma, le ou les huiles végétales H1 de par la présence de motifs d'acide gras linéaire insaturé, vont permettre de former un réseau par réactivité des insaturations, permettant de conférer au mélange une viscosité accrue, et donc les propriétés de tenue finale recherchée à la composition cosmétique. En complément, le ou les huiles végétales H2 de par l'absence de glycérol d'une part et l'absence ou la faible teneur de chaînes polyinsaturées d'autre part, vont permettre de contrôler cette viscosité et de conférer des propriétés de glissant et de non-collant en adéquation avec les besoins requis pour une composition cosmétique confortable et agréable à l'application. Dans le cas de compositions semi-solides ou solides, le ou les huiles végétales H2 vont conférer des propriétés de fermeté à la composition cosmétique.

Dans le cadre de la présente invention, le terme « fermeté » doit être compris comme la valeur de la force de compression mesurée au texturomètre et divisée par la surface de la sonde du texturomètre en contact avec la composition.

Le terme «collant » correspond à la valeur de la force qu'il faut appliquer pour extraire la sonde du texturomètre divisée par la surface de la sonde au contact de la composition. Cette valeur peut être positive, on emploie alors le terme de « collant » ou négative on emploie alors le terme de « non-collant ». Le « non-collant » peut également être évalué par un test sensoriel « in vivo ». Dans ce cas des volontaires évaluent le « non-collant » d''un article obtenu à l'aide de la composition (par exemple un gloss) en évaluant l'adhérence, c-à-d la force nécessaire pour séparer les lèvres d'une personne après application et après 3 contacts. Cela consiste à exercer 3 légers pincements avec les lèvres et à évaluer si elles collent entre elles ou non.

De manière préférée, l'huile H2 selon l'invention est substantiellement exempte de glycérol et comprend ainsi moins de 0,5 % en poids, de préférence moins de 0,1 % en poids, plus préférentiellement moins de 0,01 % en poids de glycérol, par rapport au poids total de l'huile H2.

D'une façon préférée, l'huile (H1) comprend un triglycéride constitué d'esters d'acides gras et de glycérol dont les acides gras peuvent avoir des longueur de chaînes variées de C4 à C24, cers derniers pouvant être linéaires ou ramifiés, saturés ou insaturés ; De préférence, l'huile H1 est un triglycéride constitué d'esters d'acides gras et de glycérol dont au moins deux motifs d'acide gras linéaire insaturé, au sein de ses esters d'acide gras constitutifs, sont choisis parmi l'acide oléique, l'acide linoléique, l'acide linolénique, l'acide eicosénoïque, l'acide ricinoléique, et préférentiellement comprend au moins deux motifs d'acides gras linéaire insaturé en C18.

De préférence, l'huile H1 comprend au moins un ester d'acide gras polyinsaturé choisi dans le groupe comprenant l'acide linoléique (18:2), l'acide linolénique (18:2), l'acide éléostéarique (18:3), l'acide stéaridonique (18:4), l'acide pinolénique (18:3), l'acide parinarique (18/4), l'acide eicosapentaénoïque EPA(C20:5), l'acide arachidonique (20:4), l'acide eicosatriénoïque (20:3), l'acide clupanodonique DPA (22:5), l'acide docosahexaénoïques DHA (22:6), l'acide docosapentaénoïque (22:5) et leurs mélanges.

D'une façon avantageuse, l'huile (H2) comprend :
∘ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
∘ au moins un acide gras monoinsaturé, ou
∘ au moins un diester d'alkyle dont au moins une teneur de 90% en poids d'alkyle(s) linéaire(s) monoinsaturé(s) ou saturé(s), ou
∘ leur(s) mélange(s).

Les acides gras monoinsaturés et les alcools gras monoinsaturés peuvent être choisi dans la liste des acides ou alcools monoinsaturés pouvant avoir des longueurs de chaînes variées de C4-C24, ces derniers pouvant être linéaires ou ramifiés. Parmi les acides gras mono insaturés ou les alcools gras monoinsaturés on peut citer notamment ceux en : C14 myristoléique, C16 palmitoléique, C18 oléique, C18 pétrosélinique, C20 gadoléique = eicosénoïque, C22 érucique, C22 ricinoléique (+ hydroxylé), C24 nervonique, myristoléique (14:1 n-5), palmitoléique (16:1 n-7), pétrosélinique (18:1 n-12).

Parmi les huiles esters formées à partir d'acides gras monoinsaturés ou d'alcool gras monoinsaturés, l'huile de jojoba est préférée.

Parmi les acides gras préférés, on retrouve l'acide oléique, l'acide palmitoléique et l'acide eicosénoïque. Le plus préféré étant l'acide oléique.

Parmi les diesters d'alkyle dont au moins une teneur de 90% en poids d'alkyle(s) linéaire(s) monoinsaturé(s) ou saturé(s), le dicaprylyl carbonate est préféré.

Dans un mode de réalisation avantageux, la composition cosmétique comprend en outre au moins un ingrédient choisi parmi une cire, une charge, une matière colorante, un dispersant de pigments, un antioxydant et leurs mélanges.

Selon un mode préféré de la présente invention, l'huile H2 est présente dans une quantité minimale d'au moins 10 % en poids, de préférence au moins 30 % en poids, plus préférentiellement entre 50 et 60 % en poids dans le mélange qui comprend l'huile H1 et H2 qui est prévu pour être traité au plasma. Ainsi, l'huile H2 peut être le composé majoritaire dans un mélange comprenant les huiles H1 et H2, de préférence jusqu'à 99 % en poids.

Selon un second aspect, l'invention a pour objet une composition cosmétique qui comprend :
- un mélange d'une huile H1 et d'au moins une huile H2, traité selon le procédé plasma selon l'invention, dans une teneur comprise entre 80% et 99,5% en poids de la composition, le rapport en poids entre H1 et H2 étant compris entre 1/1,5 et 1,5/1,
- un antioxydant dans une teneur comprise entre 0,2% et 0,5% en poids, par rapport au poids total de la composition,
- au moins un ingrédient choisi parmi les actifs, les colorants, les nacres, les pigments, les dispersants de pigments, les parfums, et
- la composition étant exempte de cire, de beurre et de dérivés siliconés.

Dans ce mode de réalisation particulier, la composition cosmétique comprend :
- un mélange d'une huile H1 et d'une huile H2, traité selon le procédé plasma de l'invention, dans une teneur comprise entre 95% et 99,5% en poids de la composition,
- l'huile H1 étant choisie pami le triglycéride oléique/linoléique/linolénique, et l'huile de ricin, et
- l'huile H2 étant choisie parmi les diesters d'alkyle, préférentiellement représente le dicaprylyl carbonate, et parmi les monoesters d'alkyle, préférentiellement l'huile de jojoba.

La composition cosmétique selon cette variante de réalisation est avantageusement un rouge à lèvres liquide, encore appelé laque à lèvres ou gloss. Par liquide on entend une composition fluide qui est capable de s'écouler sous son propre poids à une pression atmosphérique (760 mm de Hg), par opposition aux compositions dites solides ou semi-solides.

Selon un troisième aspect de l'invention, la composition comprend :
- un mélange d'une huile H1 et au moins deux huiles H2, traité selon le procédé plasma de l'invention, dans une teneur comprise entre 20% et 60% en poids de la composition, le rapport en poids entre H1 et les huiles H2 étant compris entre 3/2 et 1/3 ;
- au moins une cire, dans une teneur comprise entre 8% et 20% en poids de la composition ;
- au moins un antioxydant dans une teneur comprise entre 0,2 et 0,5% en poids par rapport au poids total de la composition,
- au moins un ingrédient choisis parmi les actifs, les colorants, les nacres, les pigments, les dispersants de pigments, les parfums,
- la composition étant exempte de gélifiant lipophile et de polymères structurants.

Le terme « gélifiant lipophile », désigne, dans le cadre de la présente demande, une substance capable de solidifier ou de gélatiniser l'huile présente dans la composition de l'invention.

Le terme « cire » désigne une matière grasse à changement réversible liquide/solide, ayant une température de fusion supérieure à 55°C et généralement inférieure à 110°C, qui est liquide dans les conditions de préparation de la composition et qui présente une organisation cristalline anisotrope à l'état solide. La cire peut être polaire ou apolaire

Dans cet aspect particulier de l'invention, d'une façon avantageuse, l'huile H1 est choisie parmi, l'huile de ricin, l'huile de tournesol, l'huile de camélia, le beurre de karité, l'huile d'olive, l'huile d'avocat, l'huile de noisette, l'huile de tournesol à haute teneur en acide oléique, l'huile de chia, l'huile d'hibiscus, l'huile de camélia et leurs mélanges; les huiles H2 sont préférentiellement choisies parmi l'huile de jojoba, l'acide oléique, et leurs mélanges, la cire est choisie parmi la cire d'abeille, la cire de tournesol, la cire de candellila, la cire de son de riz, la cire de carnauba, la tribehenine, ou un de leur mélange, la cire étant mélangée aux huiles H1 et H2 et soumise au traitement plasma.

De préférence, après traitement plasma des huiles H1 et H2, la cire peut être ajoutée au mélange comprenant lesdites huiles H1 et H2. Ce mode de réalisation est également illustré dans les exemples.

La composition cosmétique selon ce troisième aspect de l'invention est avantageusement un rouge à lèvres solide ou semi-solide, encore appelé stick à lèvres.

Le mélange d'huiles d'origine végétale circule en continu d'un point bas à un point haut à l'extérieur de l'enceinte. Lors de la circulation, de préférence en boucle, le mélange est contrôlé en température, pression et viscosité ; et circule un certain nombre de fois dans l'enceinte jusqu'à obtention de la viscosité souhaitée.

Lors de la circulation, le mélange d'huiles peut être filtré et échantillonné.

De manière préférée, la composition cosmétique selon l'invention peut être obtenue par un procédé qui comprend les étapes suivantes :
- Mélanger au moins une huile végétale (H1) avec au moins une huile végétale (H2) dans lequel :
- ladite au moins une huile végétale (H1) comprend :
   ∘ au moins un triglycéride constitué d'esters d'acides gras et de glycérol, ou
   ∘ au moins un ester d'acide gras polyinsaturé, ou
   ∘ leur(s) mélange(s),
- ladite au moins une huile (H2) comprend :
   ∘ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
   ∘ au moins un acide gras monoinsaturé, ou
   ∘ au moins un diester d'alkyle dont au moins une teneur de 90% en poids d'alkyle(s) linéaire(s) monoinsaturé(s) ou saturé(s), ou
   ∘ leur(s) mélange(s),
- Mise à disposition d'une enceinte comprenant une série d'électrodes, de préférence au moins 5 électrodes, pour générer un plasma dans une atmosphère sous vide, ledit plasma étant agencé pour être généré par décharge électrique, de préférence DBD, en présence d'un plasmagène qui comprend de l'hydrogène ;
- Incorporation dudit mélange dans ladite enceinte et génération du plasma en présence dudit plasmagène jusqu'à la formation par polymérisation radicalaire et réticulation d'une composition visqueuse partiellement hydrogénée qui présente une rhéologie non-newtonienne avec une viscosité dynamique à 50°C supérieure à 250 mPa.s, préférentiellement supérieure à 500 mPa.s ou supérieure à 2000 mPa.s, préférentiellement supérieure à 3000 mPa.s, et inférieure à 10 000 mPa.s ou allant de 2000 à 10000 mPa.s ou une viscosité cinématique à 50°C comprise entre 250 et 20000 mm²/sec ;
- Récupération de ladite composition cosmétique.

La présente invention concerne aussi un procédé de préparation d'une composition cosmétique caractérisé en ce qu'il comprend les étapes suivantes :
- Mélanger au moins une huile végétale (H1) avec au moins une huile végétale (H2) dans lequel
   - ladite au moins une huile végétale (H1) comprend :
      ∘ au moins un triglycéride constitué d'esters d'acides gras et de glycérol, ou
      ∘ au moins un ester d'acide gras polyinsaturé, ou
      ∘ leur(s) mélange(s), et
   - ladite au moins une huile (H2) comprend :
      ∘ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
      ∘ Au moins un acide gras monoinsaturé, ou
      ∘ Au moins un diester d'alkyle dont au moins une teneur de 90% en poids d'alkyle(s) linéaire(s) monoinsaturé(s) ou saturé(s), ou
      ∘ Leur(s) mélange(s),
- Mise à disposition d'une enceinte comprenant une série d'électrodes, de préférence au moins 10 électrodes, pour générer un plasma dans une atmosphère sous vide, ledit plasma étant agencé pour être généré par décharge électrique, de préférence DBD, en présence d'un plasmagène qui comprend de l'hydrogène ;
- Incorporation dudit mélange dans ladite enceinte et génération du plasma en présence dudit plasmagène jusqu'à la formation par polymérisation radicalaire et réticulation d'une composition visqueuse partiellement hydrogénée qui présente une rhéologie non-newtonienne avec une viscosité dynamique à 50°C supérieure à 250 mPa.s, préférentiellement supérieure à 500 mPa.s ou supérieure à 2000 mPa.s, préférentiellement supérieure à 3000 mPa.s, et inférieure à 10 000 mPa.s ou allant de 2000 et 10000 mPa.s ou une viscosité cinématique à 50°C comprise entre 250 et 20000 mm²/sec ;
- Récupération de ladite composition cosmétique ;
- optionnellement broyer les pigments en présence de dispersant de pigment, et les ajouter au mélange obtenu après traitement plasma ;
- éventuellement ajouter les ingrédients complémentaires choisis parmi les antioxydants, parfums, actifs, et les mélanger avec la composition obtenue avant ou après traitement plasma ;
- De manière avantageuse, le procédé suivant l'invention permet le soin et/ou le maquillage de matières kératiniques, en particulier des lèvres ou des ongles, et comprend l'application sur lesdites matières kératiniques, en particulier les lèvres, d'une composition cosmétique selon la présente invention.

De manière avantageuse, le procédé suivant l'invention permet le soin et/ou le maquillage de matières kératiniques, en particulier des lèvres ou des ongles, et comprend l'application sur lesdites matières kératiniques, en particulier les lèvres, d'une composition cosmétique selon la présente invention.

Toutes les caractéristiques énoncées ci-avant sont combinables entre elles.

Les compositions selon la présente demande peuvent en outre contenir au moins une ou plusieurs huiles non traitées par un procédé plasma ou solvant organique.

Par huile ou solvant organique, on entend un corps non aqueux liquide à température ambiante et pression atmosphérique. L'huile peut être volatile ou non volatile.

Par« huile ou solvant organique volatile », on entend au sens de l'invention tout milieu non aqueux susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10 3 à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par « huile non volatile », on entend une huile restant sur les matières kératiniques à température ambiante et pression atmosphérique au moins plusieurs heures et ayant notamment une pression de vapeur inférieure à 10⁻³ mm de Hg (0,13Pa).

L'huile ou solvant organique volatil ou non volatil, si elle est présente, en plus du mélange d'huile H1 et H2 issu d'un traitement plasma, peut être présente dans la composition dans une teneur allant de 0,05 à 20% de préférence 0,1 à 15% en poids par rapport au poids total de la composition. La composition selon l'invention peut comprendre des huiles volatiles et/ou des huiles non volatiles, et leurs mélanges.

Les huiles (ou solvants organiques) volatiles peuvent être des huiles hydrocarbonées, des huiles siliconées, linéaire(s), cyclique(s) ou ramifié(s), ou leurs mélanges. D'une façon avantageuse, la composition cosmétique selon l'invention est exempte d'huile siliconées, et selon un mode particulièrement intéressant de l'invention, la composition ne comprend que des huiles issues du mélange d'huile ayant subi un traitement plasma.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ d'origine naturelle ou pétrolière, comme les isoalcanes en C₈-C₁₆ tels que l'isododécane (encore appelé 2,2,4,4,6-pentaméthylheptane), l'isodécane, l'isohexadécane, les esters ramifiés en C₈-C₁₆. le néopentanoate d'iso-hexyle, et leurs mélanges.

Chacune des compositions conformes à l'invention peut également comprendre au moins une huile ou solvant organique non volatile, qui peut être en particulier choisi parmi les huiles hydrocarbonées et/ou siliconées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C4 à C24, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de luzerne, de pavot, de potimarron, de sésame, de courge, de colza, de cassis, d'onagre, de millet, d'orge, de quinoa, de seigle, de carthame, de bancoulier, de passiflore, de rosier muscat, de camélia ; ou encore les triglycérides des acides caprylique/caprique;
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters naturels comme par exemple les triglycérides, ou les dérivés d'acide pélargoniques ;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthylhexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique;
et leurs mélanges.

Un ou plusieurs agent(s) actif(s), d'origine naturelle ou synthétique, ayant une activité biologique, par exemple choisis parmi les vitamines, les oligo-éléments, l'allantoïne, les protéines végétales, les extraits végétaux, les agents hydratants, les agents antiâges, les antioxydants, les agents favorisant l'éclat et des mélanges de ceux-ci. En particulier, l'agent actif est choisi parmi une eau de fruit de vanilla planifolia, le niacinamide, l'acide hyaluronique et ses dérivés, un extrait de levure et des mélanges de ceux-ci.

Les compositions conformes à l'invention peuvent également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, l'oxychlorure de bismuth, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le 13-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30% en poids par rapport au poids total de la composition.

Les compositions conformes à l'invention peuvent également comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le mica, la silice, le kaolin, les poudres de polyamide, de poly-13-alanine et de polyéthylène, les poudres de polymères de tétrafluoroéthylène comme le Téflon ^{®}, la lauroyl-lysine, l'amidon, le nitrure de bore, les micro sphères creuses polymériques expansées telles que celles de chlorure de polyvinylidène/acrylonitrile, les poudres acryliques, les particules de polyméthacrylate de méthyle et les microbilles de résine de silicone, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

Les charges peuvent représenter de 0,1 à 25% en particulier de 0,2 à 20% en poids par rapport au poids total de la composition.

Les compositions conformes à l'invention peuvent également comprendre au moins une fibre.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur section peut être quelconque et notamment de section circulaire ou polygonale (carrée, hexagonale ou octogonale) selon l'application spécifique envisagée. Leur forme peut être linéaire, courbe, sinusoïdale ou frisée. En particulier, leurs extrémités sont épointées et/ou polies pour éviter de se blesser.

A titre de fibres utilisables dans la composition selon l'invention, on peut citer les fibres non rigides telles que les fibres de polyamide (NylonO) ou les fibres rigides telles que les fibres de polyimide-amide ou de poly-(p-phénylènetéréphtalamide) (ou d'aramide).

Les fibres peuvent être présentes dans la composition selon l'invention en une teneur allant de 0,01% à 10% en poids, par rapport au poids total de la composition, en particulier de 0,1% à 5% en poids, et plus particulièrement de 0,3% à 3% en poids

D'autres additifs habituellement utilisés en cosmétique peuvent également être présents dans la composition selon l'invention, notamment des conservateurs, des agents antioxydants, des filtres UV organiques et/ou inorganiques, ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés. Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition cosmétique peut être à destination d'un produit cosmétique choisi notamment parmi les rouges à lèvres, les gloss, les fonds de teint fluide, compact, les vernis à ongles, les ombres à paupières, les mascaras, les crayons à cils ou à sourcils, les crèmes de soins, les sérums, les produits à usage de protection solaire, les toiletries telles que les gels douches, etc...

Selon un aspect particulièrement avantageux, l'invention comprend un procédé cosmétique de soin et/ou de maquillage des matières kératiniques, en particulier des lèvres ou des ongles, comprenant l'application sur lesdites matières kératiniques, en particulier les lèvres.

Avantageusement, la présente invention concerne aussi un rouge à lèvre, un fard à paupières, un blush, un gloss ou un fond de teint comprenant une composition selon l'invention.

Selon un mode alternatif avantageux un rouge à lèvre, un fard à paupières, un blush, un gloss ou un fond de teint comprend majoritairement l'huile H2, de préférence 100 % de l'huile H2 selon l'invention.

la fermeté d'une composition est établie sur une valeur moyenne de trois mesures réalisées au texturomètre.

La fermeté de la composition, qui est exprimée en grammes (g), est déterminée par la mesure de la force de compression mesurée à 20°C à l'aide d'un texturomètre vendu sous la dénomination « TA-XT Plus Microstable System » par la société Swantech. Le texturomètre est équipé d'une sonde hémisphérique en Delrin « Hemispherical Delrin » en forme de doigt d'un diamètre de 12,7 mm se déplaçant à la vitesse de mesure de 5mm/s et pénétrant dans la composition à une profondeur de 5 mm.

La valeur de la fermeté est la force de compression mesurée, divisée par la surface de la sonde hémisphérique du texturomètre en contact avec la composition.

La mesure se fait dans des pots parfumeurs 15 ml de 40mm de hauteur et 38mm de diamètre, remplis à température ambiante jusqu'au col soit 20mm de hauteur, par la composition. Les échantillons ainsi préparés sont conservés pendant 12 h à 20°C avant d'effectuer la mesure.

Le collant (valeur positive) ou le non-collant (valeur négative) de la composition, qui est exprimée en grammes (g), est la valeur déterminée par la mesure de la force pour retirer la sonde divisée par la surface de la sonde hémisphérique du texturomètre en contact avec la composition.

La mesure se fait de façon analogue à ce qui été décrit pour la fermeté avec le même équipement dans des pots parfumeurs 15 ml de 40mm de hauteur et 38mm de diamètre, remplis à température ambiante jusqu'au col soit 20mm de hauteur, par la composition. Les échantillons ainsi préparés sont conservés pendant 12 h à 20°C avant d'effectuer la mesure.

De manière générale pour obtenir la composition cosmétique selon l'invention, le mélange d'huiles d'origine végétale est traité par plasma.

Tout 'abord et de manière exemplative, environ 3 litres du mélange d'huiles végétales est placé dans une cuve. Cette dernière est une enceinte circulaire contenant une pluralité d'électrodes (de préférence environ 5 électrodes) connectées à une source de courant alternatif. Ces électrodes sont des disques en aluminium de 25 cm de diamètre et d'une épaisseur de 2 mm. Entre ces électrodes sont placées des disques en pyrex d'un diamètre de 28 cm et d'une épaisseur de 5 mm. Sauf indication contraire, l'enceinte est ensuite remplie d'hydrogène à une pression comprise entre 130 et 260 mbar. La cuve est ensuite mise en rotation à une vitesse de 5 tours par minutes, et sauf indication contraire, une tension comprise entre 1000 et 3000 V est appliquée aux électrodes, il reste ensuite à appliquer une tension et une fréquence précises. Ces fréquences sont choisies de manière à ce que le courant de décharge soit maximal et stable. Dans ce cas-ci par exemple, des fréquences comprises entre 17 kHz et 80 kHz, de préférence entre 17 et 66 kHz peuvent être utilisées.

Lorsque l'enceinte comprend plus de 25 électrodes, plus de 30 électrodes la cuve a une forme rectangulaire.

Dans le cadre de la présente invention, il est à noter que la viscosité de la composition selon l'invention est non-newtonienne.

La viscosité cinématique indiquée dans la présente invention est déterminée selon la norme ASTM D 445. La viscosité cinématique peut être mesurée à 40 °C ou à 50 °C en fonction de la composition analysée, comme illustré dans les exemples. La viscosité cinématique est mesurée avec un appareillage de marque LAUDA iVisc équipés de capillaires Ubbelohde.

De manière préférée, la viscosité cinématique mesurée à 50°C du mélange d'au moins deux huiles végétales traitées ensemble par plasma est comprise entre 250 et 20000 mm²/sec, de préférence comprise entre 500 et 5000 mm²/sec, plus préférentiellement comprise entre 600 et 4000 mm²/sec, plus préférentiellement encore entre 700 et 3000 mm²/sec.

De manière préférée, lorsque ledit mélange comprend plus de 2 huiles, la viscosité cinématique est préférentiellement mesurée à une température de 50 °C. Le cas échéant à 40°C si la mesure peut être réalisée, par exemple lorsque le mélange est constitué de 2 huiles.

Le mélange d'au moins deux huiles végétales traitées ensemble par plasma présente une viscosité dynamique à 50°C supérieure à 250 mPa.s, préférentiellement supérieure à 500 mPa.s ou supérieure à 2000 mPa.s, préférentiellement supérieure à 3000 mPa.s, et inférieure à 10 000 mPa.s . Cette viscosité dynamique du mélange d'au moins deux huiles végétales peut être mesurée à 40 °C ou à 50 °C. La température de 50 °C sera préférée.

La viscosité dynamique du mélange d'au moins deux huiles végétales traité par plasma est mesurée à l'aide d'un viscosimètre Anton Paar muni d'un système cône-plan, CP50-0.5, selon la norme ISO 2884-1 (Détermination de la viscosité au moyen de viscosimètres rotatifs). Les mesures sont obtenues sous contrainte de cisaillement de 0 à 500 s⁻¹ en prenant 1 point toutes les 2 secondes, un maintien pendant 3 minutes à 1000 s⁻¹ et finalement de 500 à 0 s⁻¹ en prenant 1 point toutes les 2 secondes à une température de 50 °C.

Lorsque la composition comprend en outre un ingrédient de formulation choisi parmi la liste comprenant une cire, une charge, une matière colorante, un dispersant de pigments, un antioxydant et leurs mélanges pour fournir un produit cosmétique, la viscosité dynamique de la composition est comprise entre 2000 et 20000 mPa.s. Dans ce cas de figure, les mesures de viscosité dynamique sont réalisées au moyen d'un viscosimètre RM 100TOUCH CP 2000 de la société Lamy Rhéology. La viscosité est la grandeur physique qui caractérise la résistance des matériaux à l'écoulement. Un cône 40mm de diamètre, angle de 4°+plan de 40 mm est utilisé. Un cisaillement de 100s⁻¹ est utilisé. Les échantillons sont mesurés à température ambiante c'est-à-dire 25°C sans avoir été en étuve.

De manière avantageuse, il est possible de traiter un mélange d'huile constitué de 40% de polyglycérides oléique/linoléique/linolénique (Viamérime^{™}) et 60% de dicaprylyl carbonate (Cetiol CC) à une fréquence comprise entre 35 et 66 kHz durant 900 minutes jusqu'à l'obtention d'une viscosité cinématique de 6528 mm²/sec ) 40°C.

Selon un mode préférentiel, il est possible de traiter un mélange d'huile constitué de 40% de polyglycérides oléique/linoléique/linolénique (Viamérime^{™}) et 60% de dicaprylyl carbonate (Cetiol CC) à une fréquence comprise entre 35 et 66 kHz durant 1087 minutes jusqu'à l'obtention d'une viscosité cinématique de 15 000 mm²/sec à 40°C.

Le tableau 1 indique une série d'huiles végétales selon l'invention avec leur teneur en composés.

L'invention sera maintenant illustrée par les exemples non limitatifs suivants.

### Exemples

### Exemple 1 : Préparation d'un gloss transparent

**Etape 1** : traitement plasma du mélange H1 et H2 réalisé à une fréquence comprise entre 35 et 66 kHz durant 680 minutes jusqu'à l'obtention d'une viscosité cinématique de 3480 mm²/sec à 40 °C.

Un mélange comprenant 40% de polyglycérides oléique/linoléique/linolénique (Viamérime^{™}) et 60% de dicaprylyl carbonate

(Cetiol CC) est soumis à un traitement plasma selon l'invention à la fréquence donnée.

### Etape 2 : Gloss transparent

A une composition comprenant 99,1% du mélange d'huiles issu de l'étape 1 sont ajoutés 0,5% de tocopheryl acétate, 0,3% de phenoxyethanol et 0,1% de parfums.

Après agitation, une composition liquide apte à l'écoulement présentant une viscosité dynamique de 2440 mPa.s-1 est obtenue.

Les mesures de viscosité dynamique sont réalisées au moyen d'un viscosimètre RM 100 TOUCH CP 2000 de la société Lamy Rhéology. La viscosité est la grandeur physique qui caractérise la résistance des matériaux à l'écoulement. Un cône 40mm de diamètre, angle de 4°+plan de 40 mm est utilisé et un cisaillement de 100s⁻¹. Les échantillons sont mesurés à température ambiante, c'est-à-dire 25°C, sans avoir été en étuve.

### Exemple 2 : Préparation d'un gloss coloré

**Etape 1** : traitement plasma du mélange H1 et H2 réalisé à une fréquence comprise entre 35 et 66 kHz jusqu'à l'obtention d'une viscosité cinématique comprise entre 740 et 900 mm²/sec à 50 °C.

Un mélange comprenant 60% d'huile de jojoba, 28% d'huile de camélia, 10% d'huile de chia, et 2% d'huile d'hibiscus est soumis à un traitement plasma selon l'invention et à la fréquence indiquée ci-avant. Le mélange à la fin du traitement plasma présente une viscosité dynamique de 5038 mPa.s⁻¹ selon le protocole de l'étape 2 de l'exemple 1.

### Etape 2 : Gloss coloré

A une composition comprenant 88% du mélange d'huiles issu de l'étape 1 sont ajoutés un mélange comprenant les pigments décrits dans le Tableau 1, préalablement dispersés dans un dispersant. L'ensemble est agité avec une turbine défloculeuse à froid. Après 15 min l'antioxydant, le conservateur et le parfum sont ajoutés permettant d'obtenir une gloss liquide coloré conforme à l'invention. Le gloss présente une viscosité dynamique mesurée selon le protocole de l'étape 2 de l'exemple 1 de 5020 mPa.s-1.

**[Tableau 1] - Composition cosmétique de gloss coloré**

| ingrédients | fonction | % en poids total de la composition |
|---|---|---|
| Mélange d'huiles traité plasma | huiles | 88,4% |
| Pentaerythrityl adipate/caprate/caprylate/heptanoate | dispersant | 7% |
| D&C red7 | pigment | 0,83% |
| FD&C Yellow 6 | pigment | 1,4% |
| Dioxide de titane | pigment | 1,17% |
| Red iron oxide | pigment | 0,38% |
| Tocopheryle acetate | antioxydant | 0,5% |
| parfums | parfum | 0,02% |
| phenoxyethanol | conservateur | 0,3% |

Le gloss obtenu présente une très bonne tenue, comparable avec celle obtenue avec un gloss comprenant un polymère synthétique pétrosourcé.

Le gloss obtenu s'étale mieux que celui obtenu avec un gloss comprenant un polymère pétrosourcé.

Sa sensorialité est bien meilleure puisqu'il est beaucoup moins collant. Il présente également une meilleure performance d'hydratation en comparaison avec le gloss comprenant un polymère synthétique pétrosourcé.

### Exemple 3 : Composition cosmétique de rouge à lèvres stick

Une composition de Rouge à lèvres pour stick est obtenue en mélangeant les ingrédients du Tableau 2 (les % sont en poids total de la composition).

**[Tableau 2] - composition cosmétique de rouge à lèvres en stick**

| Ingrédients | | % |
|---|---|---|
| Mélange d'huiles traité plasma selon l'étape 1 de l'exemple 2 | | 46,45 |
| Eutanol G | OCTYLDODECANOL | 28,8 |
| NOMCORT HK-G | GLYCERYL BEHENATE/EICOSADIOATE | 6,5 |
| SUNFLOWER WAX | HELIANTHUS ANNUUS CERA SEED (HELIANTHUS ANNUUS (SUNFLOWER) SEED WAX) | 6,0 |
| CANDELILLA WAX CG-7 | CANDELILLA CERA (EUPHORBIA CERIFERA (CANDELILLA) WAX) & BENZYL ALCOHOL | 3,4 |
| REF RICE BRAN WAX PHC3434 | ORYZA SATIVA (RICE) BRAN WAX | 1,5 |
| BR C196619 A 30% DS ClTHROL BUHLER | CI 15850 (RED 6) and POLYGLYCERYL-3 DIISOSTEARATE | 0,15 |
| BR RED LC 328 A 35% DANS CITHROL BUHLER | D&C RED 28 AL LAKE and POLYGLYCERYL-3 DIISOSTEARATE | 1,4 |
| BR YELLOW 6 A 30% DS ClTHROL BULHER | FD&C YELLOW N°6 ALUMINIUM LAKE and POLYGLYCERYL-3 DIISOSTEARATE | 0,5 |
| BR W877 A 60% DS ClTHROL BUHLER | CI 77891 (TITANIUM DIOXIDE) & ALUMINUM HYDROXIDE and POLYGLYCERYL-3 DIISOSTEARATE | 0,1 |
| ClTHROL PG32IS-LQ-(MV) | POLYGLYCERYL-3 DIISOSTEARATE | 2,0 |
| CELLULOBEADS USF | CELLULOSE | 3,0 |
| RL2558 SA HYDRABASE 2007 | FRG00000187,C,5 | 0,1 |
| BIOXAN T 90 | TOCOPHEROL & GLYCINE SOJA (SOYBEAN) OIL | 0,1 |

La composition obtenue est facilement coulable en stick, présente une bonne tenue dans le temps sans phénomène d'exsudation. Le stick est brillant et non collant.

### Exemple 4

**Etape 1** : fournir un mélange comprenant 74 % d'huile de tournesol, 18 % d'huile de jojoba, 4 % d'huile de chia, et 4 % d'huile de camélia. Traitement par plasma du mélange réalisé à une fréquence comprise entre 17et 66 kHz jusqu'à l'obtention d'une viscosité cinématique comprise entre 800 et 900 mm²/sec à 50 °C.

Un mélange comprenant 74% d'huile de tournesol, 18% d'huile de jojoba, 4% d'huile de chia, et 4% d'huile de camelia est soumis à un traitement plasma selon l'invention et à la fréquence indiquée ci-avant. Le mélange à la fin du traitement plasma présente une viscosité dynamique de 3100 mPa.s-1 selon le protocole de l'étape 2 de l'exemple 1.

### Etape 2 : stick coloré

Une composition de Rouge à lèvres pour stick est obtenue en mélangeant les ingrédients du Tableau 3 (les % sont en poids total de la composition).

**[Tableau 3] - composition cosmétique de rouge à lèvres en stick**

| Ingrédients | | % |
|---|---|---|
| Mélange d'huiles traité plasma selon l'étape 1 de l'exemple 4 | | 75.25 |
| NOMCORT HK-G | GLYCERYL BEHENATE/EICOSADIOATE | 6,5 |
| SUNFLOWER WAX | HELIANTHUS ANNUUS CERA SEED (HELIANTHUS ANNUUS (SUNFLOWER) SEED WAX) | 6,0 |
| CANDELILLA WAX CG-7 | CANDELILLA CERA (EUPHORBIA CERIFERA (CANDELILLA) WAX) & BENZYL ALCOHOL | 3,4 |
| REF RICE BRAN WAX PHC3434 | ORYZA SATIVA (RICE) BRAN WAX | 1,5 |
| BR C196619 A 30% DS ClTHROL BUHLER | CI 15850 (RED 6) and POLYGLYCERYL-3 DIISOSTEARATE | 0,15 |
| BR RED LC 328 A 35% DANS CITHROL BUHLER | D&C RED 28 AL LAKE and POLYGLYCERYL-3 DIISOSTEARATE | 1,4 |
| BR YELLOW 6 A 30% DS ClTHROL BULHER | FD&C YELLOW N°6 ALUMINIUM LAKE and POLYGLYCERYL-3 DIISOSTEARATE | 0,5 |
| BR W877 A 60% DS ClTHROL BUHLER | CI 77891 (TITANIUM DIOXIDE) & ALUMINUM HYDROXIDE and POLYGLYCERYL-3 DIISOSTEARATE | 0,1 |
| ClTHROL PG32IS-LQ-(MV) | POLYGLYCERYL-3 DIISOSTEARATE | 2,0 |
| CELLULOBEADS USF | CELLULOSE | 3,0 |
| RL2558 SA HYDRABASE 2007 | FRG00000187,C,5 | 0,1 |
| BIOXAN T 90 | TOCOPHEROL & GLYCINE SOJA (SOYBEAN) OIL | 0,1 |

La composition obtenue est facilement coulable en stick, présente une bonne tenue dans le temps sans phénomène d'exsudation. Le stick est brillant et non collant.

### Exemple 5

**Etape 1** : fournir un mélange comprenant 90 % d'acide oléique, et 10 % d'huile de chia. Traitement par plasma du mélange réalisé à une fréquence comprise entre 40 et 70 kHz jusqu'à l'obtention d'une viscosité cinématique comprise entre 720 et 880 mm²/sec à 50 °C.

Un mélange comprenant 90 % d'acide oléique, et 10 % d'huile de chia est soumis à un traitement plasma selon l'invention et à la fréquence indiquée ci-avant. Le mélange à la fin du traitement plasma présente une fermeté de 1545 g force au texturomètre.

La fermeté est établie sur une valeur moyenne de trois mesures réalisées au texturomètre.

La fermeté de la composition, qui est exprimée en grammes (g), est déterminée par la mesure de la force de compression mesurée à 20°C à l'aide d'un texturomètre vendu sous la dénomination « TA-XT Plus Microstable System » par la société Swantech. Le texturomètre est équipé d'une sonde hémisphérique en Delrin « Hemispherical Delrin » en forme de doigt d'un diamètre de 12,7 mm se déplaçant à la vitesse de mesure de 5mm/s et pénétrant dans la composition à une profondeur de 5 mm.

La valeur de la fermeté est la force de compression mesurée, divisée par la surface de la sonde hémisphérique du texturomètre en contact avec la composition.

La mesure se fait dans des pots parfumeurs 15 ml de 40mm de hauteur et 38mm de diamètre, remplis à température ambiante jusqu'au col soit 20mm de hauteur, par la composition. Les échantillons ainsi préparés sont conservés pendant 12 h à 20°C avant d'effectuer la mesure.

### Etape 2 : stick coloré

Une composition de Rouge à lèvres pour stick est obtenue en mélangeant les ingrédients du Tableau 4 (les % sont en poids total de la composition).

**[Tableau 4] - composition cosmétique de rouge à lèvres en stick**

| Ingrédients | | % |
|---|---|---|
| Mélange d'huiles traité plasma selon l'étape 1 de l'exemple 5 | | 25 |
| Eutanol G | OCTYLDODECANOL | 56,75 |
| SUNFLOWER WAX | HELIANTHUS ANNUUS CERA SEED (HELIANTHUS ANNUUS (SUNFLOWER) SEED WAX) | 6,0 |
| CANDELILLA WAX CG-7 | CANDELILLA CERA (EUPHORBIA CERIFERA (CANDELILLA) WAX) & BENZYL ALCOHOL | 3,4 |
| REF RICE BRAN WAX PHC3434 | ORYZA SATIVA (RICE) BRAN WAX | 1,5 |
| BR C196619 A 30% DS ClTHROL BUHLER | CI 15850 (RED 6) and POLYGLYCERYL-3 DIISOSTEARATE | 0,15 |
| BR RED LC 328 A 35% DANS CITHROL BUHLER | D&C RED 28 AL LAKE and POLYGLYCERYL-3 DIISOSTEARATE | 1,4 |
| BR YELLOW 6 A 30% DS ClTHROL BULHER | FD&C YELLOW N°6 ALUMINIUM LAKE and POLYGLYCERYL-3 DIISOSTEARATE | 0,5 |
| BR W877 A 60% DS ClTHROL BUHLER | CI 77891 (TITANIUM DIOXIDE) & ALUMINUM HYDROXIDE and POLYGLYCERYL-3 DIISOSTEARATE | 0,1 |
| ClTHROL PG32IS-LQ-(MV) | POLYGLYCERYL-3 DIISOSTEARATE | 2,0 |
| CELLULOBEADS USF | CELLULOSE | 3,0 |
| RL2558 SA HYDRABASE 2007 | FRG00000187,C,5 | 0,1 |
| BIOXAN T 90 | TOCOPHEROL & GLYCINE SOJA (SOYBEAN) OIL | 0,1 |

La composition obtenue est facilement coulable en stick, présente une bonne tenue dans le temps sans phénomène d'exsudation. Ce stick est brillant et non collant.

### Exemple 6 : Préparation d'un gloss coloré

**Etape 1** : fournir un mélange comprenant 40 % d'acide oléique (huile H2), 52% d'huile de tournesol (huile H1) 4 % d'huile de chia (huile H1), 4% d'huile de camelia (huile H1). Traitement par plasma du mélange réalisé à une fréquence comprise entre 40 et 70 kHz jusqu'à l'obtention d'une viscosité cinématique comprise entre 720 et 880 mm²/sec à 50 °C.

Un mélange comprenant 40 % d'acide oléique (huile H2), 52% d'huile de tournesol (huile H1) 4 % d'huile de chia (huile H1), 4% d'huile de camelia (huile H1) est soumis à un traitement plasma selon l'invention et à la fréquence indiquée ci-avant. Le mélange à la fin du traitement plasma présente une viscosité dynamique de 4200 mPa.s⁻¹ selon le protocole de l'étape 2 de l'exemple 1.

### Etape 2 : Gloss coloré

A une composition comprenant 88% du mélange d'huiles issu de l'étape 1 sont ajoutés un mélange comprenant les pigments décrits dans le Tableau 1, préalablement dispersés dans un dispersant. L'ensemble est agité avec une turbine défloculeuse à froid. Après 15 min l'antioxydant, le conservateur et le parfum sont ajoutés permettant d'obtenir une gloss liquide coloré conforme à l'invention. Le gloss présente une viscosité dynamique mesurée selon le protocole de l'étape 2 de l'exemple 1 de 4150 mPa.s-1.

**[Tableau 1] - Composition cosmétique de gloss coloré**

| ingrédients | fonction | % en poids total de la composition |
|---|---|---|
| Mélange d'huiles traité plasma | huiles | 88,4% |
| Pentaerythrityl adipate/caprate/caprylate/heptanoate | dispersant | 7% |
| D&C red7 | pigment | 0,83% |
| FD&C Yellow 6 | pigment | 1,4% |
| Dioxide de titane | pigment | 1,17% |
| Red iron oxide | pigment | 0,38% |
| Tocopheryle acetate | antioxydant | 0,5% |
| parfums | parfum | 0,02% |
| phenoxyethanol | conservateur | 0,3% |

Le gloss obtenu est brillant et non-collant. Il présente l'avantage de bien suspendre les pigments sans ajout de gélifiant.

## Revendications

1. Composition cosmétique **caractérisée en ce qu'**elle comprend :
- un mélange d'au moins deux huiles végétales traitées ensemble selon un procédé plasma dans une teneur comprise entre 20% et 99% en poids, par rapport au poids total de la composition,
- ledit mélange présentant une viscosité dynamique à 50 °C supérieure à 250 mPa.s, préférentiellement supérieure à 500 mPa.s ou supérieure à 2000 mPa.s, préférentiellement supérieure à 3000 mPa.s, et inférieure à 10 000 mPa.s,
- les au moins deux huiles végétales étant choisies parmi :
- au moins une huile végétale (H1) comprenant :
∘ au moins un triglycéride constitué d'esters d'acides gras et de glycerol, ou
∘ au moins un ester d'acide gras polyinsaturé, ou
∘ leur(s) mélange(s), et
- au moins une huile (H2) comprenant :
∘ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
∘ Au moins un acide gras monoinsaturé, ou
∘ Au moins un diester d'alkyle dont au moins une teneur de 90% en poids d'alkyle(s) linéaire(s) monoinsaturé(s) ou saturé(s), ou
∘ Leur(s) mélange(s),

2. Composition cosmétique selon la revendication 1 **caractérisée en ce que** l'huile (H1) comprend au moins deux motifs d'acide gras linéaire insaturé, au sein de ses esters d'acide gras constitutifs, choisis parmi l'acide oléique, l'acide linoléique, l'acide linolénique, l'acide eicosénoïque, l'acide ricinoléique, préférentiellement comprend au moins deux motifs d'acides gras linéaire insaturé en C18.

3. Composition selon la revendication 1, **caractérisée en ce que** l'huile (H1) comprend au moins un ester d'acide gras polyinsaturé choisi dans le groupe comprenant l'acide linoléique (18:2), l'acide linolénique (18:2), l'acide éléostéarique (18:3), l'acide stéaridonique (18:4), l'acide pinolénique (18:3), l'acide parinarique (18/4), l'acide eicosapentaénoïque EPA(C20:5), l'acide arachidonique (20:4), l'acide eicosatriénoïque (20:3), l'acide clupanodonique DPA (22:5), l'acide docosahexaénoïques DHA (22:6), l'acide docosapentaénoïque (22:5) et leurs mélanges.

4. Composition cosmétique selon l'une quelconque des revendications 1 à 3, **caractérisée en ce que** l'huile (H2) est choisie dans la liste des acides gras mono insaturés, de préférence les acides gras suivants : C14 myristoléique, C16 palmitoléique, C18 oléique, C18 pétrosélinique, C20 gadoléique = eicosénoique, C22 érucique, C22 ricinoléique (+ hydroxylé), C24 nervonique, myristoléique (14:1 n-5), palmitoléique (16:1 n-7), pétrosélinique (18:1 n-12).

5. Composition cosmétique selon l'une quelconque des revendications 1 à 4 **caractérisée en ce que** l'huile H2 est l'huile de jojoba.

6. Composition cosmétique selon l'une quelconque des revendications précédentes **caractérisée en ce qu'**elle comprend en outre un ingrédient choisi parmi une cire, une charge, une matière colorante, un dispersant de pigments, un antioxydant et leurs mélanges.

7. Composition selon l'une quelconque des revendications précédentes, **caractérisée** en ce l'huile H2 est présente dans une quantité minimale d'au moins 10 % en poids, de préférence au moins 30 % en poids, plus préférentiellement entre 50 et 60 % en poids dans le mélange qui comprend l'huile H1 et H2 qui est prévu pour être traité au plasma, de préférence l'huile H2 est le composé majoritaire dans le mélange comprenant les huiles H1 et H2, de préférence l'huile H2 est présente dans une quantité allant jusqu'à 99 % en poids, par rapport au poids total dudit mélange.

8. Composition cosmétique selon l'une quelconque des revendications précédentes **caractérisée en ce qu'**elle comprend :
• un mélange d'une huile H1 et d'au moins une huile H2, traité selon le procédé plasma selon l'invention, dans une teneur comprise entre 80% et 99,5% en poids de la composition, le rapport en poids entre H1 et H2 étant compris entre 1/1,5 et 1,5/1
• un antioxydant dans une teneur comprise entre 0,2 et 0,5% en poids, par rapport au poids total de la composition, et
• au moins un ingrédient choisis parmi les actifs, les colorants, les nacres, les pigments, les dispersants de pigments, les parfums,
• la composition étant exempte de cire, de beurre et de dérivés siliconés.

9. Composition cosmétique selon la revendication 8 **caractérisée en ce qu'**elle comprend :
• un mélange d'une huile H1 et d'une huile H2, traité selon le procédé plasma de l'invention, dans une teneur comprise entre 95% et 99,5% en poids de la composition,
• l'huile H1 étant choisie par le triglycéride oléique/linoléique/linolénique, l'huile de ricin, et
• l'huile H2 étant choisie parmi l'huile de jojoba, les diesters d'alkyle, préférentiellement représente le dicaprylyl carbonate, ou leur(s) mélange(s).

10. Composition cosmétique selon l'une des revendications précédentes, **caractérisée en ce qu'**elle comprend :
• un mélange d'une huile H1 et au moins deux huiles H2, traité selon le procédé plasma de l'invention, dans une teneur comprise entre 20% et 60% en poids de la composition, le rapport en poids entre H1 et les huiles H2 étant compris entre 3/2 et 1/3 ;
• au moins une cire, dans une teneur comprise entre 8% et 20% en poids de la composition
• au moins un antioxydant dans une teneur comprise entre 0,2 et 0,5% en poids par rapport au poids total de la composition,
• au moins un ingrédient choisis parmi les actifs, les colorants, les nacres, les pigments, les dispersants de pigments, les parfums,
• la composition étant exempte de gélifiant et de polymères structurants.

11. Composition cosmétique selon la revendication 10 **caractérisée en ce que** l'huile H1 est choisie parmi l'huile de ricin, l'huile de chia, l'huile d'hibiscus, et l'huile de camélia, et l'huile H2 est l'huile de jojoba, la cire est choisie parmi la cire d'abeille, la cire de tournesol, la cire de candellila, la cire de son de riz, la cire de carnauba, la tribehenine, ou un de leur mélange, la cire étant mélangée aux huiles H1 et H2 et soumise au traitement plasma.

12. Composition cosmétique selon l'une quelconque des revendications précédentes, **caractérisée en ce que** l'huile H1 est choisie parmi l'huile de ricin, l'huile de tournesol, l'huile de camélia, le beurre de karité, l'huile d'olive, l'huile d'avocat, l'huile de noisette, l'huile de tournesol à haute teneur en acide oléique l'huile de chia, l'huile d'hibiscus, l'huile de camélia ou leurs mélanges ; les huiles H2 sont préférentiellement choisies parmi l'huile de jojoba, l'acide oléique, et leurs mélanges, la cire est choisie parmi la cire d'abeille, la cire de tournesol, la cire de candellila, la cire de son de riz, la cire de carnauba, la tribehenine, ou leur mélange.

13. Composition cosmétique selon l'une quelconque des revendications précédentes pouvant être obtenue par un procédé qui comprend les étapes suivantes :
- Mélanger au moins une huile végétale (H1) avec au moins une huile végétale (H2) dans lequel :
- ladite au moins une huile végétale (H1) comprend :
∘ au moins un triglycéride constitué d'esters d'acides gras et de glycérol, ou
∘ au moins un ester d'acide gras polyinsaturé, ou
∘ leur(s) mélange(s),
- ladite au moins une huile (H2) comprend :
∘ au moins un monoester d'acide gras monoinsaturé formé par la réaction d'un acide gras monoinsaturé et d'un alcool gras saturé ou formé par la réaction d'un acide gras saturé et d'un alcool gras monoinsaturé, ou
∘ au moins un acide gras monoinsaturé, ou
∘ au moins un diester d'alkyle dont au moins une teneur de 90% en poids d'alkyle(s) linéaire(s) monoinsaturé(s) ou saturé(s), ou leur(s) mélange(s)
- Mise à disposition d'une enceinte comprenant une série d'électrodes, de préférence au moins 10 électrodes, pour générer un plasma dans une atmosphère sous vide, ledit plasma étant agencé pour être généré par décharge électrique, de préférence DBD, en présence d'un plasmagène qui comprend de l'hydrogène ;
- Incorporation dudit mélange dans ladite enceinte et génération du plasma en présence dudit plasmagène jusqu'à la formation par polymérisation radicalaire et réticulation d'une composition visqueuse partiellement hydrogénée qui présente une rhéologie non-newtonienne avec une viscosité dynamique supérieure à 250 mPa.s, préférentiellement supérieure à 500 mPa.s ou supérieure à 1000 mPa.s ou supérieure à 1500 mPA.s ou supérieure à 2000 mPa.s, préférentiellement supérieure à 3000 mPa.s, et inférieure à 10 000 mPa.s ou allant de 2000 à 10000 mPa.s ;
- Récupération de ladite composition cosmétique.

14. Procédé cosmétique de soin et/ou de maquillage des matières kératiniques, en particulier des lèvres ou des ongles, comprenant l'application sur lesdites matières kératiniques, en particulier les lèvres, d'une composition selon l'une des revendications précédentes.

15. Rouge à lèvre, fard à paupières, blush, gloss ou fond de teint comprenant la composition selon l'une des revendications 1 à 13.
